Europäisches Patentamt

European Patent Office

Office européen des brevets

(11) Publication number: **0 553 298 B1**

# EUROPEAN PATENT SPECIFICATION

(45) Date of publication of patent specification: **17.11.94**    (51) Int. Cl.5: **A61K 9/12**

(21) Application number: **92900942.1**

(22) Date of filing: **09.10.91**

(86) International application number:
**PCT/US91/07574**

(87) International publication number:
**WO 92/06675 (30.04.92 92/10)**

(54) **AEROSOL FORMULATION COMPRISING BECLOMETHASONE 17,21 DIPROPIONATE.**

(30) Priority: **18.10.90 US 599694**

(43) Date of publication of application:
**04.08.93 Bulletin  93/31**

(45) Publication of the grant of the patent:
**17.11.94 Bulletin  94/46**

(84) Designated Contracting States:
**AT BE CH DE DK ES FR GB IT LI LU NL SE**

(56) References cited:
**WO-A-86/03750**
**WO-A-91/11173**
**WO-A-91/11495**

(73) Proprietor: **MINNESOTA MINING AND MANU-**
**FACTURING COMPANY**
**3M Center,**
**P.O. Box 33427**
**St. Paul, Minnesota 55133-3427 (US)**

(72) Inventor: **SCHULTZ, Robert, K.**
**Post Office Box 33427**
**Saint Paul, MN 55133-3427 (US)**
Inventor: **SCHULTZ, David, W.**
**Post Office Box 33427**
**Saint Paul, MN 55133-3427 (US)**

(74) Representative: **Bowman, Paul Alan et al**
**LLOYD WISE, TREGEAR & CO.**
**Norman House**
**105-109 Strand**
**London WC2R OAE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

TECHNICAL FIELD OF THE INVENTION

This invention pertains to solution aerosol formulations suitable for use in administering drugs. In another aspect this invention pertains to formulations comprising beclomethasone 17,21 dipropionate.

BACKGROUND OF THE INVENTION

Pharmaceutical suspension aerosol formulations currently use a mixture of liquid chlorofluorocarbons as the propellant. Fluorotrichloromethane, dichlorodifluoromethane and dichlorotetrafluoroethane are the most commonly used propellants in aerosol formulations for administration by inhalation.

Chlorofluorocarbons have been implicated in the destruction of the ozone layer and their production is being phased out. Hydrofluorocarbon 134a (HFC-134a, 1,1,1,2-tetrafluoroethane) and hydrofluorocarbon 227 (HFC-227, 1,1,1,2,3,3,3-heptafluoropropane) are viewed as being less destructive to ozone than many chlorofluorocarbon propellants; furthermore, they have low toxicity and vapor pressure suitable for use in aerosols.

Beclomethasone 17,21 dipropionate is commercially available as an aerosol product comprising a suspension of a chlorofluorohydrocarbon solvate of beclomethasone 17,21 dipropionate in chlorofluorohydrocarbon propellants. Preparation of the solvate requires several processing Steps and is required in order to obtain a stable aerosol formulation, i.e., one in which the micronized particles of active ingredient remain in the desired respirable particle size range. A solution formulation of beclomethasone 17,21 dipropionate could simplify formulation manufacture and increase the respirable fraction (i.e., the percentage of active ingredient able to reach the airways of the lung where the pharmaceutical effect is exerted).

U.S. Pat. No. 2,868,691 discloses a self-propelling pharmaceutical aerosol formulation comprising i) a medicament; ii) a propellant represented generally by the formula $C_mH_nC1_yF_z$, wherein m is an integer less than 3, n is an integer or zero, y is an integer or zero, and z is an integer, such that n + y + z = 2m + 2; and iii) a cosolvent which assists in the dissolution of the medicament in the propellant. Ethanol is an example of a cosolvent disclosed in this patent. The above formula representing the propellant component generically embraces HFC-134a. This patent does not, however, disclose beclomethasone 17,21 dipropionate or suggest how stable solution aerosol formulations (i.e., formulations that are chemically stable and exhibit desirable respirable fraction) containing any propellant and beclomethasone 17,21 dipropionate can be prepared.

European Patent Publication No. 0372777 discloses a self-propelling aerosol formulation which may be free from CFC's which comprises a medicament, 1,1,1,2-tetrafluoroethane, a surface active agent and at least one compound having a higher polarity than 1,1,1,2-tetrafluoroethane. Examples 10 to 12 disclose solution formulations comprising beclomethasone 17,21 dipropionate (0.005g), surface active agent (0.006g), (sorbitan trioleate, oleic acid and lecithin in Examples 10 to 12 respectively), ethanol (1.350g) and 1,1,1,2-tetrafluoroethane (4.040g).

SUMMARY OF THE INVENTION

The present invention provides an aerosol formulation comprising a therapeutically effective amount of beclomethasone 17,21 dipropionate, a propellant comprising a hydrofluorocarbon selected from the group consisting of 1,1,1,2-tetrafluoroethane, 1,1,1,2,3,3,3-heptafluoropropane, and a mixture thereof, and ethanol in an amount effective to solubilize the beclomethasone 17,21 dipropionate in the propellant, the formulation being further characterized in that Substantially all of the beclomethasone 17,21 dipropionate is dissolved in the formulation, and that the formulation contains no more than 0.0005% by weight of any surfactant.

Certain of the preferred formulations of the invention exhibit very desirable chemical stability and provide respirable fractions significantly greater than commercially available beclomethasone 17,21 dipropionate products. Moreover, the formulations of the invention are convenient to manufacture since no solvate of the active ingredient need be prepared.

The pharmaceutical solution aerosol formulations of the invention are suitable for pulmonary, buccal, or nasal administration.

2

## DETAILED DESCRIPTION OF THE INVENTION

All weight percentages recited herein are based on the total weight of the formulation unless otherwise indicated.

The medicament beclomethasone 17,21 dipropionate is generally present in a formulation of the invention in a therapeutically effective amount, i.e., an amount such that one or more metered volumes of the formulation contains an amount of drug effective to exert the intended therapeutic action. Preferably the medicament will constitute about 0.02 to about 0.6 percent by weight, more preferably about 0.05 to about 0.5 percent by weight of the total weight of the formulation.

Ethanol is generally present in an amount effective to solubilize the beclomethasone 17,21 dipropionate in the propellant. Preferably, ethanol constitutes about 1 to about 20 percent by weight of the total weight of the aerosol formulation. More preferably, ethanol constitutes about 2 to about 12 percent by weight and even more preferably about 2 to about 10 percent by weight of the aerosol formulation. Most preferably, ethanol will be present in an amount sufficient to dissolve substantially all of the medicament present in the formulation and to maintain the medicament dissolved over the time period and conditions experienced by commercial aerosol products, but not substantially in excess of said amount. Particularly desirable formulations of the invention, while not containing amounts of ethanol substantially in excess of that required (during manufacture of the formulation) to dissolve the amount of active ingredient employed, may be subjected to a temperature of -20°C. without precipitation of the active ingredient.

The hydrofluorocarbon propellant can be HFC-134a, HFC-227, or a mixture thereof. The propellant preferably constitutes from about 80 to about 99 percent by weight, preferably from about 88 to about 98 percent by weight, and more preferably about 90 to about 98 percent by weight of the total weight of the aerosol formulation. The hydrofluorocarbon propellant is preferably the only propellant present in the formulations of the invention. However, one or more other propellants (e.g., 1-chloro-1,1-difluoroethane) can also be present.

The formulations of the invention are substantially free of any surfactant. By "substantially free" as used in the instant specification and claims is meant that the formulations contain no more than 0.0005 percent by weight of a surfactant based on the total weight of the formulation. Preferred formulations contain no surfactant. Presence of a significant amount of a surfactant is believed to be undesirable in the case of solution formulations of beclomethasone 17,21 dipropionate because surfactants such as oleic acid and lecithin seem to promote chemical degradation of the active ingredient when the latter is dissolved in the mixture of HFC-134a and ethanol.

Preferred formulations according to the invention consist essentially of beclomethasone 17,21 dipropionate in an amount of about 0.05 to about 0.35 percent by weight based on the weight of the total formulation, ethanol in an amount of about 2 to about 8 percent by weight based on the total weight of the formulation, and 1,1,1,2-tetrafluoroethane.

The solution formulations of the invention can be prepared by dissolving the desired amount of beclomethasone 17,21 dipropionate in the desired amount of anhydrous ethanol accompanied by stirring or sonication. The aerosol vial may then be filled using conventional cold-fill or pressure-fill methods.

The following examples are provided to illustrate the invention but should not be construed as limiting the invention.

## Examples 1 - 7

Formulations containing the following ingredients (TABLE I) in the indicated amounts were prepared with the percentages being expressed in parts by weight based upon the total weight of the particular formulation. The active ingredient employed in preparing the formulations of Examples 2, 3, and 5 - 7 was beclomethasone dipropionate, USP while that employed in preparing the formulations of Examples 1 and 4 was a conventional trichloromonofluoromethane solvate of beclomethasone dipropionate. The formulations of Examples 1, 4, 5 and 6 were prepared by i) dissolving the active ingredient in the ethanol; ii) metering the solution obtained above into an aluminum vial and crimping a continuous valve onto the vial; iii) pressure-filling the vial with 1,1,1,2-tetrafluoroethane; iv) chilling the vial to -60°C.; and v) replacing the continuous valve with a 50 microliter valve which is available under the trade designation "W303-98" from 3M. The formulations of Examples 2, 3 and 7 were prepared by i) dissolving the active ingredient in the ethanol; ii) metering the solution obtained above into an aluminum vial and crimping a 50 microliter pressure-fill valve which is available under the trade designation Spraymiser™ M3652 from 3M onto the vial; and iii) pressure-filling the vial with 1,1,1,2-tetrafluoroethane.

The actuator employed in the case of all the formulations was a solution actuator available under the trade designation "M3756" from 3M. The elastomer employed in the valves in the case of all formulations was that available under the trade designation "DB-218" from American Gasket and Rubber Co. (Chicago, IL.)

TABLE I

| Ingredient | Example | | | | | | |
|---|---|---|---|---|---|---|---|
| | 1 | 2 | 3 | 4 | 5 | 6 | 7 |
| Beclomethasone 17,21 Dipropionate | 0.1% | 0.1% | 0.25% | 0.3% | 0.4% | 0.44% | 0.5% |
| Ethanol (anhydrous) | 3% | 5% | 10% | 5% | 10% | 10% | 15% |
| 1,1,1,2-Tetrafluoroethane | 96.9% | 94.9% | 89.75% | 94.7% | 89.6% | 89.56% | 84.5% |

The chemical stability of the formulation of Example 4 was determined in respect to recovery of the active ingredient over time when the formulation was stored at 40°C. TABLE II contains the data.

TABLE II

| Storage Time (Weeks) | 0 | 2 | 4 | 7 | 12 |
|---|---|---|---|---|---|
| % Recovery | 101.4, 98.7 | 101.9, 101.6 | 100.8, 99.6 | 99.3, 95.5 | 100.6 102.6 |

The formulation of Example 1 did not exhibit precipitation of the active ingredient on freezing to -60°C.

The respirable fraction provided by the formulations of Examples 1 - 7 was determined using an Anderson MK II Cascade Impactor with the average respirable fraction obtained from each being in excess of 40%. In the case of the formulations of Examples 1 and 4, the respirable fraction was about 76% and about 70%, respectively.

From the above data, it is believed that the optimum amount of active ingredient for low and high strength products would be about 0.08 and 0.34 percent by weight, respectively, based on the total weight of the formulations.

Example 8

A mixture containing 1.67 g of beclomethasone 17,21 dipropionate and 160g of cold (-65°C) ethanol was homogenized using a Virtis 45 homogenizer. The resulting suspension was placed in a one gallon stainless steel filling vessel equipped with a stir bar. A 1839 g portion of cold (-65°C) 1,1,1,2-tetrafluoroethane was added to the filling vessel. After about 5 minutes of stirring, a solution was obtained. The resulting formulation contained 0.08 percent by weight of beclomethasone 17,21 dipropionate, 8.0 percent by weight of ethanol and 91.92 percent by weight of 1,1,1,2-tetrafluoroethane. The formulation was cold filled into aerosol vials and then 50 µL cold fill valves were crimped onto the vials.

Example 9

Using the general method of Example 8, a formulation containing 0.34 percent by weight of beclomethasone 17,21 dipropionate, 8.0 percent by weight of ethanol and 91.66 percent by weight of 1,1,1,2-tetrafluoroethane was prepared. The formulation was cold filled as a suspension into aerosol vials which were then equipped with 50 µL cold fill valves. The formulation changed from a suspension to a solution as the vials warmed to room temperature.

Example 10

A formulation containing 0.3 percent by weight of beclomethasone 17,21 dipropionate, 10 percent by weight of ethanol and 89.7 percent by weight of 1,1,1,2,3,3,3-heptafluoropropane was prepared by i) weighing a 30 mg portion of beclomethasone 17,21 dipropionate into an aerosol vial ii) crimping a continuous valve onto the vial and iii) pressure filling with a solution containing 10 percent ethanol in 1,1,1,2,3,3,3-heptafluoropropane.

**Claims**
**Claims for the following Contracting States : AT, BE, CH, DE, DK, FR, GB, IT, NL, SE**

1. An aerosol formulation comprising a therapeutically effective amount of beclomethasone 17,21 dipropionate, a propellant comprising a hydrofluorocarbon selected from the group consisting of 1,1,1,2-tetrafluoroethane, 1,1,1,2,3,3,3-heptafluoropropane, and a mixture thereof, and ethanol in an amount effective to solubilize the beclomethasone 17,21 dipropionate in the propellant, the formulation being further characterized in that substantially all of the beclomethasone 17,21 dipropionate is dissolved in the formulation, and that the formulation contains no more than 0.0005% by weight of any surfactant.

2. A solution aerosol formulation according to Claim 1, comprising between 0.02 and about 0.6 percent by weight beclomethasone 17,21 dipropionate, between about 1 and about 20 percent by weight ethanol, and between about 80 and about 99 percent by weight of said propellant.

3. A solution aerosol formulation according to Claim 1 wherein said beclomethasone 17,21 dipropionate is present in an amount of about 0.05 to about 0.5 percent by weight.

4. A solution aerosol formulation according to Claim 1 wherein said ethanol is present in an amount of about 2 to about 12 percent by weight.

5. A solution aerosol formulation according to Claim 1 wherein said ethanol is present in an amount of about 2 to 10 percent by weight.

6. A solution aerosol formulation according to Claim 1 wherein said propellant is present in an amount of about 88 to about 98 percent by weight.

7. A solution aerosol formulation according to Claim 1 comprising 1,1,1,2-tetrafluoroethane as substantially the only propellant.

8. A solution aerosol formulation according to Claim 1 comprising 1,1,1,2,3,3,3-heptafluoropropane as substantially the only propellant.

9. A solution aerosol formulation according to Claim 1 comprising beclomethasone 17,21 dipropionate in an amount of about 0.05 to about 0.5 percent by weight, ethanol in an amount of about 2 to about 12 percent by weight and said propellant in an amount of about 88 to about 98 percent by weight.

10. A solution aerosol formulation according to Claim 1 comprising beclomethasone 17, 21 dipropionate in an amount of about 0.05 to about 0.45 percent by weight, ethanol in an amount of about 2 to about 10 percent by weight and said propellant in an amount of about 90 to about 98 percent by weight.

11. A solution aerosol formulation according to Claim 1, consisting essentially of beclomethasone 17,21 dipropionate in an amount of about 0.05 to about 0.35 percent by weight, ethanol in an amount of about 2 to about 8 percent by weight, and 1,1,1,2-tetrafluoroethane.

12. A solution aerosol formulation according to Claim 1, wherein the amount of ethanol present is not substantially in excess of the amount required to dissolve substantially all of the beclomethasone 17,21 dipropionate, but is sufficient to permit said formulation to be subjected to a temperature of -20°C. without significant precipitation of said beclomethasone 17, 21 dipropionate.

13. A method of preparing a solution aerosol formulation comprising the step of combining a therapeutically effective amount of beclomethasone 17,21 dipropionate, a propellant selected from the group consisting of 1,1,1,2-tetrafluoroethane, 1,1,1,2,3,3,3-heptafluoropropane, and a mixture thereof, and an amount of ethanol effective to solubilize the beclomethasone 17,21 dipropionate in the propellant.

**Claims for the following Contracting State : ES**

1.  A process for preparing an aerosol formulation which comprises combining a therapeutically effective amount of beclomethasone 17,21 dipropionate, a propellant comprising a hydrofluorocarbon selected from the group consisting of 1,1,1,2-tetrafluoroethane, 1,1,1,2,3,3,3-heptafluoropropane, and a mixture thereof, and ethanol in an amount effective to solubilize the beclomethasone 17,21 dipropionate in the propellant, such that substantially all of the beclomethasone 17,21 dipropionate is dissolved in the formulation, the formulation containing no more than 0.0005% by weight of any surfactant.

2.  A process as claimed in Claim 1, comprising combining between 0.02 and about 0.6 percent by weight beclomethasone 17,21 dipropionate, between about 1 and 20 percent by weight ethanol, and between about 80 and about 99 percent by weight of said propellant.

3.  A process as claimed in Claim 1 or Claim 2 in which the beclomethasone 17,21 dipropionate is present in an amount of about 0.05 to about 0.5 percent by weight.

4.  A process as claimed in any preceding Claim in which the ethanol is present in an amount of about 2 to about 12 percent by weight.

5.  A process as claimed in Claim 4 in which the ethanol is present in an amount of about 2 to 10 percent by weight.

6.  A process as claimed in any preceding Claim in which the propellant is present in an amount of about 88 to about 98 percent by weight.

7.  A process as claimed in any preceding Claim comprising 1,1,1,2-tetrafluoroethane as substantially the only propellant.

8.  A process as claimed in any one of Claims 1 to 6 comprising 1,1,1,2,3,3,3-heptafluoropropane as substantially the only propellant.

9.  A process as claimed in Claim 1 comprising combining beclomethasone 17,21 dipropionate in an amount of about 0.05 to about 0.5 percent by weight, ethanol in an amount of about 2 to about 12 percent by weight and said propellant in an amount of about 88 to about 98 percent by weight.

10. A process as claimed in Claim 1 comprising combining beclomethasone 17,21 dipropionate in an amount of about 0.05 to about 0.45 percent by weight, ethanol in an amount of about 2 to about 10 percent by weight and said propellant in an amount of about 90 to about 98 percent by weight.

11. A process as claimed in Claim 1 comprising combining beclomethasone 17,21 dipropionate in an amount of about 0.05 to about 0.35 percent by weight, ethanol in an amount of about 2 to about 8 percent by weight, and 1,1,1,2-tetrafluoroethane.

12. A process as claimed in Claim 1 wherein the amount of ethanol present is not substantially in excess of the amount required to dissolve substantially all of the beclomethasone 17,21 dipropionate, but is sufficient to permit said formulation to be subjected to a temperature of -20°C without significant precipitation of said beclomethasone 17,21 dipropionate.

**Patentansprüche**

**Patentansprüche für folgende Vertragsstaaten : AT, BE, CH, DE, DK, FR, GB, IT, NL, SE**

1.  Aerosolformulierung, umfassend eine therapeutisch wirksame Menge von Beclomethason-17,21-dipropionat, ein Treibgas, umfassend einen Fluorkohlenwasserstoff, ausgewählt aus der Gruppe 1,1,1,2-Tetrafluorethan, 1,1,1,2,3,3,3-Heptafluorpropan und einem Gemisch davon, und Ethanol in wirksamer Menge, um das Beclomethason-17,21-dipropionat im Treibgas löslich zu machen, wobei die Formulierung weiter dadurch gekennzeichnet ist, daß im wesentlichen das ganze Beclomethason-17,21-dipropionat in der Formulierung gelöst ist, und daß die Formulierung nicht mehr als 0,0005 Gew.-% irgendeines grenzflächenaktiven Mittels enthält.

6

2. Lösungsaerosolformulierung nach Anspruch 1, umfassend zwischen 0,02 und etwa 0,6 Gew.-% Beclomethason-17,21-dipropionat, zwischen etwa 1 und etwa 20 Gew.-% Ethanol und zwischen etwa 80 und etwa 99 Gew.-% des Treibgases.

3. Lösungsaerosolformulierung nach Anspruch 1, in der das Beclomethason-17,21-dipropionat in einer Menge von etwa 0,05 bis etwa 0,5 Gew.-% vorhanden ist.

4. Lösungsaerosolformulierung nach Anspruch 1, in der das Ethanol in einer Menge von etwa 2 bis etwa 12 Gew.-% vorhanden ist.

5. Lösungsaerosolformulierung nach Anspruch 1, in der das Ethanol in einer Menge von etwa 2 bis 10 Gew.-% vorhanden ist.

6. Lösungsaerosolformulierung nach Anspruch 1, in der das Treibgas in einer Menge von etwa 88 bis etwa 98 Gew.-% vorhanden ist.

7. Lösungsaerosolformulierung nach Anspruch 1, umfassend 1,1,1,2-Tetrafluorethan als im wesentlichen einziges Treibgas.

8. Lösungsaerosolformulierung nach Anspruch 1, umfassend 1,1,1,2,3,3,3-Heptafluorpropan als im wesentlichen einziges Treibgas.

9. Lösungsaerosolformulierung nach Anspruch 1, umfassend Beclomethason-17,21-dipropionat in einer Menge von etwa 0,05 bis etwa 0,5 Gew.-%, Ethanol in einer Menge von etwa 2 bis etwa 12 Gew.-% und das Treibgas in einer Menge von etwa 88 bis etwa 98 Gew.-%.

10. Lösungsaerosolformulierung nach Anspruch 1, umfassend Beclomethason-17,21-dipropionat in einer Menge von etwa 0,05 bis etwa 0,45 Gew.-%, Ethanol in einer Menge von etwa 2 bis etwa 10 Gew.-% und das Treibgas in einer Menge von etwa 90 bis etwa 98 Gew.-%.

11. Lösungsaerosolformulierung nach Anspruch 1, im wesentlichen bestehend aus Beclomethason-17,21-dipropionat in einer Menge von etwa 0,05 bis etwa 0,35 Gew.-%, Ethanol in einer Menge von etwa 2 bis etwa 8 Gew.-%, und 1,1,1,2-Tetrafluorethan.

12. Lösungsaerosolformulierung nach Anspruch 1, in der die Menge an vorhandenem Ethanol nicht wesentlich im Überschuß über die erforderliche Menge ist, um im wesentlichen das gesamte Beclomethason-17,21-dipropionat zu lösen, aber ausreicht, daß die Formulierung auf eine Temperatur von -20°C abgekühlt werden kann, ohne daß eine deutliche Niederschlagsbildung des Beclomethason-17,21-dipropionats auftritt.

13. Verfahren zur Herstellung einer Lösungsaerosolformulierung, umfassend den Schritt des Zusammenbringens einer therapeutisch wirksamen Menge von Beclomethason-17,21-dipropionat, eines Treibgases, ausgewählt aus der Gruppe, bestehend aus 1,1,1,2-Tetrafluorethan, 1,1,1,2,3,3,3-Heptafluorpropan und einem Gemisch davon, und einer Menge an Ethanol, die wirksam ist, um das Beclomethason-17,21-dipropionat im Treibgas löslich zu machen.

**Patentansprüche für folgenden Vertragsstaat : ES**

1. Verfahren zur Herstellung einer Aerosolformulierung, umfassend das Zusammenbringen einer therapeutisch wirksamen Menge von Beclomethason-17,21-dipropionat, eines Treibgases, umfassend einen Fluorkohlenwasserstoff, ausgewählt aus der Gruppe 1,1,1,2-Tetrafluorethan, 1,1,1,2,3,3,3-Heptafluorpropan und einem Gemisch davon, und Ethanol in wirksamer Menge, um das Beclomethason-17,21-dipropionat im Treibgas löslich zu machen, sodaß im wesentlichen das ganze Beclomethason-17,21-dipropionat in der Formulierung gelöst ist, und die Formulierung nicht mehr als 0,0005 Gew.-% irgendeines grenzflächenaktiven Mittels enthält.

2. Verfahren nach Anspruch 1, umfassend das Zusammenbringen von zwischen 0,02 und etwa 0,6 Gew.-% Beclomethason-17,21-dipropionat, zwischen etwa 1 und 20 Gew.-% Ethanol und zwischen etwa 80 und etwa 99 Gew.-% des Treibgases.

3. Verfahren nach Anspruch 1 oder Anspruch 2, wobei das Beclomethason-17,21-dipropionat in einer Menge von etwa 0,05 bis etwa 0,5 Gew.-% vorhanden ist.

4. Verfahren nach einem der vorstehenden Ansprüche, wobei das Ethanol in einer Menge von etwa 2 bis etwa 12 Gew.-% vorhanden ist.

5. Verfahren nach Anspruch 4, wobei das Ethanol in einer Menge von etwa 2 bis 10 Gew.-% vorhanden ist.

6. Verfahren nach einem der vorstehenden Ansprüche, wobei das Treibgas in einer Menge von etwa 88 bis etwa 98 Gew.-% vorhanden ist.

7. Verfahren nach einem der vorstehenden Ansprüche, umfassend 1,1,1,2-Tetrafluorethan als im wesentlichen einziges Treibgas.

8. Verfahren nach einem der Ansprüche 1 bis 6, umfassend 1,1,1,2,3,3,3-Heptafluorpropan als im wesentlichen einziges Treibgas.

9. Verfahren nach Anspruch 1, umfassend das Zusammenbringen von Beclomethason-17,21-dipropionat in einer Menge von etwa 0,05 bis etwa 0,5 Gew.-%, Ethanol in einer Menge von etwa 2 bis etwa 12 Gew.-% und dem Treibgas in einer Menge von etwa 88 bis etwa 98 Gew.-%.

10. Verfahren nach Anspruch 1, umfassend das Zusammenbringen von Beclomethason-17,21-dipropionat in einer Menge von etwa 0,05 bis etwa 0,45 Gew.-%, Ethanol in einer Menge von etwa 2 bis etwa 10 Gew.-% und dem Treibgas in einer Menge von etwa 90 bis etwa 98 Gew.-%.

11. Verfahren nach Anspruch 1, umfassend das Zusammenbringen von Beclomethason-17,21-dipropionat in einer Menge von etwa 0,05 bis etwa 0,35 Gew.-%, Ethanol in einer Menge von etwa 2 bis etwa 8 Gew.-%, und 1,1,1,2-Tetrafluorethan.

12. Verfahren nach Anspruch 1, wobei die Menge an vorhandenem Ethanol nicht wesentlich im Überschuß über die erforderliche Menge ist, um im wesentlichen das gesamte Beclomethason-17,21-dipropionat zu lösen, aber ausreicht, daß die Formulierung auf eine Temperatur von -20°C abgekühlt werden kann, ohne daß eine deutliche Niederschlagsbildung des Beclomethason-17,21-dipropionats auftritt.

**Revendications**
**Revendications pour les Etats contractants suivants : AT, BE, CH, DE, DK, FR, GB, IT, NL, SE**

1. Formulation d'aérosol comprenant une quantité thérapeutiquement efficace de 17,21-dipropionate de beclométhasone, un agent de propulsion comprenant un hydrofluorocarbone choisi dans le groupe comprenant le 1,1,1,2-tétrafluoroéthane, le 1,1,1,2,3,3,3-heptafluoropropane et un mélange de ceux-ci, et de l'éthanol en une quantité efficace pour solubiliser le 17,21-dipropionate de beclométhasone dans l'agent de propulsion, la formulation étant de plus caractérisée en ce que essentiellement la totalité du 17,21-dipropionate de beclométhasone est dissoute dans la formulation et en ce que la formulation ne contient pas plus de 0,0005 % en poids d'agent tensioactif.

2. Formulation d'aérosol en solution suivant la revendication 1, comprenant entre 0,02 et environ 0,6 % en poids de 17,21-dipropionate de beclométhasone, entre environ 1 et environ 20 % en poids d'éthanol et entre environ 80 et environ 99 % en poids de l'agent de propulsion susdit.

3. Formulation d'aérosol en solution suivant la revendication 1, dans laquelle le 17,21-dipropionate de beclométhasone est présent en une quantité d'environ 0,05 à environ 0,5 % en poids.

4. Formulation d'aérosol en solution suivant la revendication 1, dans laquelle l'éthanol est présent en une quantité d'environ 2 à environ 12 % en poids.

5. Formulation d'aérosol en solution suivant la revendication 1, dans laquelle l'éthanol est présent en une quantité d'environ 2 à 10 % en poids.

6. Formulation d'aérosol en solution suivant la revendication 1, dans laquelle l'agent de propulsion est présent en une quantité d'environ 88 à environ 98 % en poids.

7. Formulation d'aérosol en solution suivant la revendication 1, comprenant du 1,1,1,2-tétrafluoroéthane comme essentiellement le seul agent de propulsion.

8. Formulation d'aérosol en solution suivant la revendication 1, comprenant du 1,1,1,2,3,3,3-heptafluoropropane comme essentiellement le seul agent de propulsion.

9. Formulation d'aérosol en solution suivant la revendication 1, comprenant du 17,21-dipropionate de beclométhasone en une quantité d'environ 0,05 à environ 0,5 % en poids, de l'éthanol en une quantité d'environ 2 à environ 12 % en poids et l'agent de propulsion en une quantité d'environ 88 à environ 98 % en poids.

10. Formulation d'aérosol en solution suivant la revendication 1, comprenant du 17,21-dipropionate de beclométhasone en une quantité d'environ 0,05 à environ 0,45 % en poids, de l'éthanol en une quantité d'environ 2 à environ 10 % en poids et l'agent de propulsion en une quantité d'environ 90 à environ 98 % en poids.

11. Formulation d'aérosol en solution suivant la revendication 1, se composant essentiellement de 17,21-dipropionate de beclométhasone en une quantité d'environ 0,05 à environ 0,35 % en poids, d'éthanol en une quantité d'environ 2 à environ 8 % en poids et de 1,1,1,2-tétrafluoroéthane.

12. Formulation d'aérosol en solution suivant la revendication 1, dans laquelle la quantité d'éthanol présente n'est pas sensiblement en excès de la quantité requise pour dissoudre essentiellement la totalité du 17,21-dipropionate de beclométhasone mais est suffisante pour permettre à la formulation d'être soumise à une température de -20°C sans une précipitation importante du 17,21-dipropionate de beclométhasone.

13. Procédé de préparation d'une formulation d'aérosol en solution comprenant l'étape consistant à combiner une quantité thérapeutiquement efficace de 17,21-dipropionate de beclométhasone, d'un agent de propulsion choisi dans le groupe comprenant le 1,1,1,2-tétrafluoroéthane, le 1,1,1,2,3,3,3-heptafluoropropane et un mélange de ceux-ci, et d'une quantité d'éthanol efficace pour solubiliser le 17,21-dipropionate de beclométhasone dans l'agent de propulsion.

**Revendications pour l'Etat contractant suivant : ES**

1. Procédé de préparation d'une formulation d'aérosol, qui comprend la combinaison d'une quantité thérapeutiquement efficace de 17,21-dipropionate de beclométhasone, d'un agent de propulsion comprenant un hydrofluorocarbone choisi dans le groupe comprenant le 1,1,1,2-tétrafluoroéthane, le 1,1,1,2,3,3,3-heptafluoropropane et un mélange de ceux-ci, et d'éthanol en une quantité efficace pour solubiliser le 17,21-dipropionate de beclométhasone dans l'agent de propulsion, de telle sorte que essentiellement la totalité du 17,21-dipropionate de beclométhasone soit dissoute dans la formulation, la formulation ne contenant pas plus de 0,0005 % en poids d'agent tensioactif.

2. Procédé suivant la revendication 1, comprenant la combinaison d'entre 0,02 et environ 0,6 % en poids de 17,21-dipropionate de beclométhasone, d'entre environ 1 et 20 % en poids d'éthanol et d'entre environ 80 et environ 99 % en poids de l'agent de propulsion.

3. Procédé suivant l'une ou l'autre des revendications 1 et 2, dans lequel le 17,21-dipropionate de beclométhasone est présent en une quantité d'environ 0,05 à environ 0,5 % en poids.

4. Procédé suivant l'une quelconque des revendications précédentes, dans lequel l'éthanol est présent en une quantité d'environ 2 à environ 12 % en poids.

**5.** Procédé suivant la revendication 4, dans lequel l'éthanol est présent en une quantité d'environ 2 à 10 % en poids.

**6.** Procédé suivant l'une quelconque des revendications précédentes, dans lequel l'agent de propulsion est présent en une quantité d'environ 88 à 98 % en poids.

**7.** Procédé suivant l'une quelconque des revendications précédentes, comprenant du 1,1,1,2-tétrafluoroéthane essentiellement comme seul agent de propulsion.

**8.** Procédé suivant l'une quelconque des revendications 1 à 6, comprenant du 1,1,1,2,3,3,3-heptafluoropropane essentiellement comme seul agent de propulsion.

**9.** Procédé suivant la revendication 1, comprenant la combinaison de 17,21-dipropionate de beclométhasone en une quantité d'environ 0,05 à environ 0,5 % en poids, d'éthanol en une quantité d'environ 2 à environ 12 % en poids et de l'agent de propulsion en une quantité d'environ 88 à environ 98 % en poids.

**10.** Procédé suivant la revendication 1, comprenant la combinaison de 17,21-dipropionate de beclométhasone en une quantité d'environ 0,05 à environ 0,45 % en poids, d'éthanol en une quantité d'environ 2 à environ 10 % en poids et de l'agent de propulsion en une quantité d'environ 90 à environ 98 % en poids.

**11.** Procédé suivant la revendication 1, comprenant la combinaison de 17,21-dipropionate de beclométhasone en une quantité d'environ 0,05 à environ 0,35 % en poids, d'éthanol en une quantité d'environ 2 à environ 8 % en poids et de 1,1,1,2-tétrafluoroéthane.

**12.** Procédé suivant la revendication 1, dans lequel la quantité d'éthanol présent n'est pas sensiblement excédentaire à la quantité requise pour dissoudre essentiellement la totalité du 17,21-dipropionate de beclométhasone mais est suffisante pour permettre à la formulation susdite d'être soumise à une température de -20 °C sans une précipitation importante du 17,21-dipropionate de beclométhasone.